# FASCICULE DE BREVET EUROPEEN

(11) **EP 0 434 523 B1**
(45) Date de publication et mention de la délivrance du brevet: **16.06.1993**
(21) Numéro de dépôt: 90403598.7
(22) Date de dépôt: 14.12.1990
(51) Int. Cl.: A61L 9/01

(54) **Désodorisation des lisiers au moyen d'ester d'acide undécylénique**
Deodorierung von Güllen mit Hilfe von Undecylensäureester
Deodorization of manures with the help of undecylenic acid ester

(30) Priorité: 19.12.1989 FR 8916793
(43) Date de publication de la demande: 26.06.1991
(73) Titulaire: DELTA AGRO INDUSTRIES, F-75015 Paris (FR); ELF ATOCHEM S.A., 92800 Puteaux (FR)
(72) Inventeur: Menassa, Aimé, Paris 16éme (FR); Caupin, Henri, F-78000 Versailles (FR)
(74) Mandataire: Rochet, Michel

(56) Documents cités:
- Eléments de la technique relevés: néant.

## Description

L'invention a pour objet une substance désodorisante pour lisiers, en particulier des lisiers de porc.

L'agriculture intensive est une source importante de nuisances, le secteur des productions animales, avec les déjections, en étant une composante non négligeable. Le cheptel porcin génère à lui-seul des quantités importantes de lisier qui, outre les problèmes de pollution occasionnés, sont des sources de nuisances par les odeurs tant au niveau de l'élevage (collecte) qu'au niveau du stockage de leur traitement et de leur épandage.

Plusieurs systèmes de désodorisation sont mis en oeuvre. Ces systèmes ont pour vocation de réduire l'odeur, mais aussi les composantes polluantes.

Tous les élevages ne font pas appel à ces moyens; la plupart procèdent à un épandage après stockage.

Parmi les techniques de désodorisation, le ruissellement par lit bactérien n'est pratiquement plus utilisé et trois systèmes prédominent: l'aération de surface, l'insufflation d'air, la méthanisation.

L'invention concerne plus précisément une substance absorbante d'odeurs de lisiers, caractérisée en ce que ladite substance comprend un ester polyoxyalkylénique d'acide undécylénique.

Parmi ces produits, l'invention concerne tout particulièrement les esters de polyoxyéthylène, polyoxypropylène et poly(oxyéthylène)(oxypropylène) dudit acide undécylénique. Elle concerne encore plus précisément les esters polyoxyalkyléniques renfermant de 2 à 10 motifs oxyalkylène.

La substance absorbante conforme à l'invention peut être constituée d'un seul des produits précités ou d'un mélange desdits produits, ce ou ces produits pouvant eux-mêmes être mis en oeuvre tels quels ou sous forme de solution ou suspension, ou encore sous forme adsorbée sur un support tel que par exemple des particules d'argile.

D'une manière générale, les esters undécyléniques sont efficaces à très faible dose, par exemple de l'ordre de 0,01 et 1 % par rapport au poids de la masse à traiter.

On peut naturellement former des compositions comprenant le ou les esters undécyléniques précités et des additifs tels que des agents bactériostatiques ou fongicides et l'acide undécylénique lui-même.

L'incorporation de l'ester précité se traduit généralement par la formation, à la surface de la matière à traiter, d'un film supprimant l'émanation d'odeur.

Les courbes annexées et qui constituent des exemples d'illustration non limitatifs, montrent l'efficacité de certains esters polyoxyéthyléniques d'acide undécylénique.

Dans ces courbes, l'axe des abscisses représente le taux d'incorporation de l'ester undécylénique, l'axe des ordonnées représentant le degré de perception olfactive de l'odeur, les valeurs 1 à 6 signifiant respectivement : néant, très faible, faible, moyen, fort et très fort.

Sur ces figures, la courbe supérieure correspond à l'odeur du lisier lui-même, la courbe inférieure correspondant à l'odeur de l'ester.

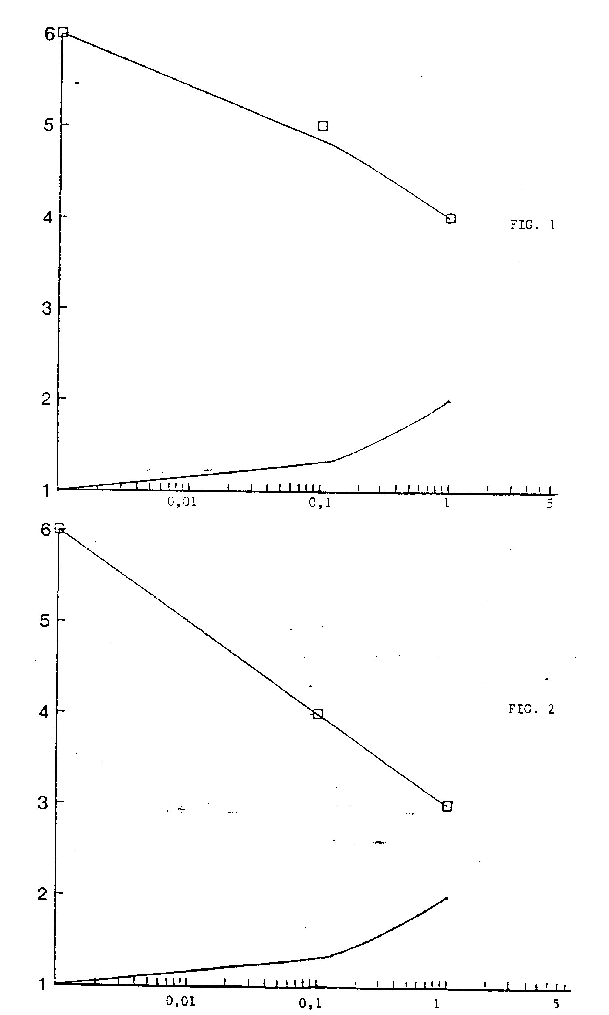

Les courbes des figures 1 et 2 qui correspondent respectivement aux esters polyoxyéthyléniques d'acide undécylénique à 8 et 10 motifs oxyéthylène, conformes à l'invention, montrent que la réduction de la perception de l'odeur du lisier n'est pas remplacée de manière sensible par la perception d'une odeur liée à l'ester lui-même.

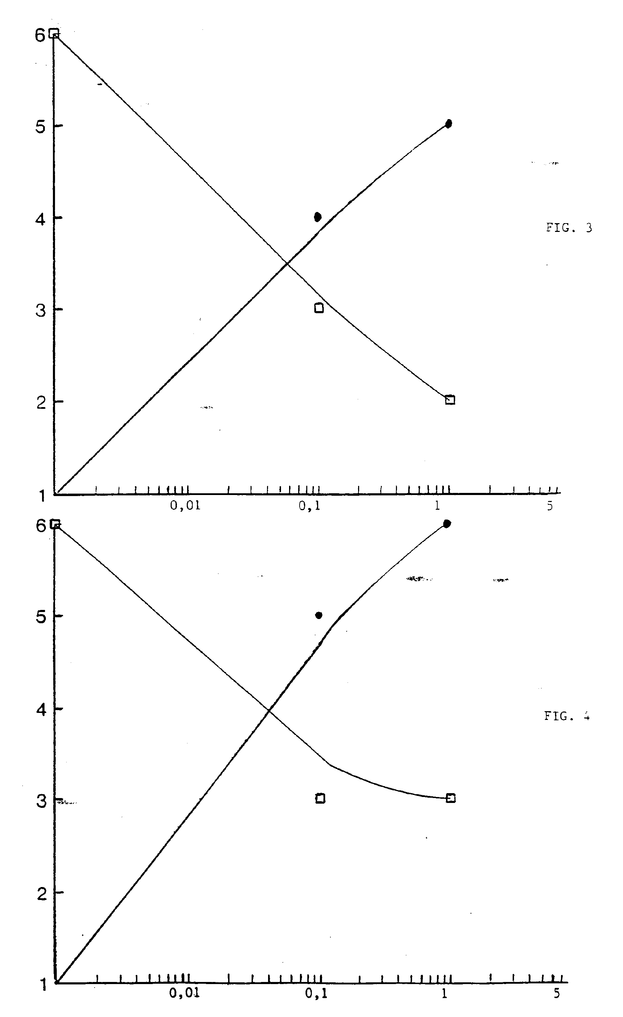

A titre de comparaison, les figures 3 et 4 montrent qu'avec respectivement l'undécylénate de méthyle et l'ester polyoxyéthylénique à 12 motifs oxyéthylène, la réduction de la perception de l'odeur du lisier est remplacée par une forte perception de l'odeur de l'ester undécylénique.

## Revendications

1. Désodorisant pour lisier,, caractérisé en ce qu'il comprend un ester polyoxyalkylénique d'acide undécylénique renfermant 2 à 10 motifs polyoxyalkylène.

2. Désodorisant pour lisier, caractérisé en ce qu'il comprend une substance selon la revendication 1 et un fongicide et/ou l'acide undécylénique.

## Patentansprüche

1. Luftverbesserer für Jauche, dadurch gekennzeichnet, daß er einen Polyoxyalkylenester der Undecylensäure mit 2 bis 10 Polyoxyalkyleneinheiten enthält.

2. Luftverbesserer für Jauche, dadurch gekennzeichnet, daß er eine Substanz gemäß Anspruch 1 und ein Fungizid und/oder Undecylensäure enthält.

## Claims

1. Deodorant for liquid manure, characterised in that it comprises a polyoxyalkylene ester of undecylenic acid containing 2 to 10 polyoxyalkylene units.

2. Deodorant for liquid manure, characterised in that it comprises a substance according to Claim 1 and a fungicide and/or undecylenic acid.
